# EUROPEAN PATENT SPECIFICATION

(11) **EP 1 254 944 B1**
(45) Date of publication and mention of the grant of the patent: **28.03.2007**
(21) Application number: 00987720.0
(22) Date of filing: 22.12.2000
(51) Int. Cl.: C09K 15/16, A61K 47/18, A61K 47/26, A61K 38/00, A61K 38/21, A61P 31/12, A61P 37/02, A61P 7/06

(54) **DRY COMPOSITIONS CONTAINING HYDROPHOBIC AMINO ACID**
HYDROPHOBE AMINOSÄUREN ENTHALTENDE TROCKENE ZUSAMMENSETZUNG
COMPOSITIONS SECHES CONTENANT UN ACIDE AMINE HYDROPHOBE

(30) Priority: 24.12.1999 JP 36805399
(43) Date of publication of application: 06.11.2002
(73) Proprietor: OTSUKA PHARMACEUTICAL CO., LTD., Chiyoda-ku Tokyo 101-8535 (JP)
(72) Inventor: YAMASHITA, Chikamasa, Naruto-shi, Tokushima 772-00 34 (JP); ODOMI, Masaaki, Itano-gun, Tokushima 771-1240 (JP)
(74) Representative: HOFFMANN EITLE
(86) International application number: PCT/JP2000/009163
(87) International publication number: WO 2001/048117

(56) References cited:
- EP-A1- 0 090 356
- WO-A-95/31479
- WO-A-96/32149
- WO-A-97/23239
- WO-A1-00/51629
- WO-A1-97/23239
- US-A- 4 623 717

## Description

### TECHNICAL FIELD

The present invention relates to a method for reducing and/or preventing coloration caused by a combined use of saccharide and amino acid. Specifically, the invention provides, as an amino acid usable with a saccharide, an amino acid having an anti-coloring effect when combined with a saccharide. The invention also provides a method for preventing coloration achieved by using the amino acid.

The invention further relates to a method for stabilizing a dry composition containing a pharmacologically active proteinaceous substance. Specifically, the invention relates to a dry composition containing a pharmacologically active proteinaceous substance to which a saccharide and an amino acid are added.

### BACKGROUND ART

In pharmaceutical compositions, particularly in dry compositions containing a pharmacologically active proteinaceous substance as an active ingredient, sucrose, glucose, fructose, maltose and like saccharides (Japanese Unexamined Patent Publications Nos. 1983-92619, 1985-48933 and 1990-138222, etc.), glycine, alanine and like amino acids (Japanese Unexamined Patent Publications No. 1983-146504) have generally been used as stabilizers effective on the active ingredient.

However, it is generally known that when these saccharides, which are used as stabilizers for pharmacologically active proteinaceous substances, are used with an amino acid, coloration caused by the Maillard reaction, etc. results. Therefore, pharmaceutical compositions containing a saccharide and an amino acid have the drawback of coloration with the passage of time.

An object of the present invention is to provide an amino acid having an anti-coloring effect for reducing and/or preventing coloration caused by the combined use of saccharide and amino acid. Specifically, the invention provides an anti-coloring agent containing the amino acid as an essential ingredient suitable for preventing the coloration caused by the change with the passage of time of a dry composition containing a saccharide, and a method for preventing coloration where the amino acid is used.

Another object of the invention is to provide an amino acid useable as a stabilizer for a dry composition containing a saccharide, particularly, a proteinaceous dry composition containing a saccharide. More specifically, the invention provides a dry composition containing a saccharide and the amino acid.

### DISCLOSURE OF THE INVENTION

The inventor has conducted extensive research to achieve the above objects. Consequently, the inventor has found that, in dry compositions containing a saccharide, specifically, dry compositions containing a saccharide and a pharmacologically active proteinaceous substance, use of specific amino acid enables the significant reduction and/or prevention of coloration attributable to saccharide. He has also found that dry compositions containing a saccharide as a stabilizer, even dry compositions containing a pharmacologically active proteinaceous substance, can further be improved in stability by adding the specific amino acid. The invention has been accomplished based on the above findings.

In other words, the invention firstly relates to use of a hydrophobic amino acid preventing the coloration caused by the combined use of saccharide and amino acid, specifically, the following embodiments are included.

### 1. Method for preventing coloration

Item 1. A method for preventing coloration of a dry composition containing a saccharide comprising adding a hydrophobic amino acid to the dry composition containing a saccharide, as defined in claim 1.

Item 2. The method for preventing coloration according to item 1, wherein the hydrophobic amino acid has a hydropathy index of not less than 3.8.

Item 3. The method for preventing coloration according to item 1, wherein the hydrophobic amino acid has a hydropathy index in the range of from 3.8 to 4.5.

Item 4. The method for preventing coloration according to item 1, wherein the hydrophobic amino acid is at least one member selected from the group consisting of valine, leucine, isoleucine and phenylalanine.

Item 5. The method for preventing coloration according to item 1, wherein the saccharide is at least one member selected from the group consisting of reducing sugars, sucrose, trehalose, mannitol and dextran 40.

Item 6. The method for preventing coloration according to item 1, wherein the hydrophobic amino acid is used in such a manner that its concentration becomes in the range of from 0.1 wt.% to 70 wt.% per 100 wt.% of the total weight of the dry composition.

Item 7. The method for preventing coloration according to item 1, wherein the dry composition is a freeze-dried composition comprising a pharmacologically active proteinaceous substance as an active ingredient.

Item 8. The method for preventing coloration according to item 20, wherein the pharmacologically active proteinaceous substance is at least one member selected from the group consisting of antiviral polypeptides, immunomodulator polypeptides and hematinic polypeptides.

Item 9. The method for preventing coloration according to item 1, wherein the pharmacologically active proteinaceous substance is interferon.

### 2. Use of hydrophobic amino acid

Item 10. Use of a hydrophobic amino acid for preventing coloration of a dry composition containing a saccharide, as defined in claim 19.

Item 11. The use of a hydrophobic amino acid according to item 10, wherein the hydrophobic amino acid has a hydropathy index of not less than 3.8.

Item 12. The use of a hydrophobic amino acid according to item 10, wherein the hydrophobic amino acid has a hydropathy index in the range of from 3.8 to 4.5.

Item 13. The use of a hydrophobic amino acid according to item 10, wherein the hydrophobic amino acid is at least one member selected from the group consisting of valine, leucine, isoleucine and phenylalanine.

Item 14. The use of a hydrophobic amino acid according to item 10, wherein the saccharide is at least one member selected from the group consisting of reducing sugars, sucrose, trehalose, mannitol and dextran 40.

Item 15. The use of a hydrophobic amino acid according to item 10, wherein the concentration of the hydrophobic amino acid is in the range of from 0.1 wt.% to 70 wt.% per 100 wt.% of the total weight of the dry composition.

Item 16. The use of a hydrophobic amino acid according to item 10, wherein the dry composition is a freeze-dried composition containing a pharmacologically active proteinaceous substance as an active ingredient.

Item 17. The use of a hydrophobic amino acid according to item 10, wherein the pharmacologically active proteinaceous substance is at least one member selected from the group consisting of antiviral polypeptides, immunomodulator polypeptides and hematinic polypeptides.

Item 18. The use of a hydrophobic amino acid according to item 10, wherein the pharmacologically active proteinaceous substance is interferon.

The present invention can effectively reduce or prevent the coloration caused by the change with the passage of time of dry compositions containing a saccharide, especially compositions containing a saccharide and a pharmacologically active proteinaceous substance. Thereby, it can provide dry compositions excellent in stability.

Secondly, the invention relates to dry compositions containing a pharmacologically active proteinaceous substance in a stable manner including the prevention of coloring.

### 3. Dry compositions

Item 19. A dry composition containing a pharmacologically active proteinaceous substance whereto a saccharide and a hydrophobic amino acid are added as a stabilizer, as defined in claim 10.

Item 20. The dry composition containing a pharmacologically active proteinaceous substance according to item 19, wherein the hydrophobic amino acid has a hydropathy index of not less than 3.8.

Item 21. The dry composition containing a pharmacologically active proteinaceous substance according to item 19, wherein the hydrophobic amino acid has a hydropathy index in the range of from 3.8 to 4.5.

Item 22. The dry composition containing a pharmacologically active proteinaceous substance according to item 19, wherein the hydrophobic amino acid is at least one member selected from the group consisting of valine, leucine, isoleucine and phenylalanine.

Item 23. The dry composition containing a pharmacologically active proteinaceous substance according to item 19, wherein the saccharide is at least one member selected from the group consisting of reducing sugars, sucrose, trehalose, mannitol and dextran 40.

Item 24. The dry composition containing a pharmacologically active proteinaceous substance according to item 19, wherein the concentration of the hydrophobic amino acid is in the range of from 0.1 wt.% to 70 wt.% per 100 wt.% of the total weight of the dry composition.

Item 25. The dry composition containing a pharmacologically active proteinaceous substance according to item 19, which is a freeze-dried composition.

Item 26. The dry composition containing a pharmacologically active proteinaceous substance according to item 19, wherein the pharmacologically active proteinaceous substance is at least one member selected from the group consisting of antiviral polypeptides, immunomodulator polypeptides and hematinic polypeptides.

Item 27. The dry composition containing a pharmacologically active proteinaceous substance according to item 19, wherein the pharmacologically active proteinaceous substance is interferon.

The present invention can provide, by combining a saccharide with a hydrophobic amino acid, dry compositions containing a pharmacologically active proteinaceous substance as an active ingredient in a stable condition even after completion of drying (freeze-drying, etc.) and during storage.

### BEST MODE FOR CARRYING OUT THE INVENTION

The method of the invention uses a hydrophobic amino acid as an essential ingredient. The method of the invention significantly prevents coloration with the passage of time, namely when used with a dry composition containing a saccharide.

The hydrophobic amino acids used in the invention are those having a hydropathy index of about 2 or larger (Jack Kyte and Russel F. Doolittel, "A Simple Method for Displaying the Hydropathic Character of a Protein." ***J. Mol. Biol**.* 157(1982): 105-132). Any amino acids having the above property may be used in the invention whether or not they are protein-constituting amino acids. The hydrophobic amino acids may preferably be those having a hydropathy index of about 2.8 or larger such as valine, leucine, isoleucine and phenylalanin; more preferably those having a hydropathy index of about 3.8 or larger; and even more preferably those having a hydropathy index in the range of from about 3.8 to about 4.5 such as valine, leucine and isoleucine.

The hydrophobic amino acids to be used in the invention may be in the form of dipeptides, tripeptides, salts or amides. Examples of the dipeptides of hydrophobic amino acids are leucyl-valine, isoleucyl-valine, isoleucyl-leucine, leucyl-glycine, etc. Examples of the tripeptides of hydrophobic amino acids are isoleucyl-leucyl-valine, leucyl-glicyl-glycine, etc. The salts of hydrophobic amino acids include salts thereof with sodium, potassium and like alkali metals or with calcium, magnesium and like alkaline earth metals; adduct salts thereof with phosphoric acid, hydrochloric acid, hydrobromic acid and like inorganic acids or with organic acids such as a sulfonic acid, etc. More specific examples of the salts of hydrophobic amino acids are an L-leucic amide hydrochloride, L-isoleucyl-β-naphthylamide hydrobromide, L-valine-β-naphthylamide, etc.

These hydrophobic amino acids (including those in a form of a salt or amide) may be used singularly or in an arbitrary combination.

The method of the invention targets saccharides generally known to cause coloration with the passage of time due to the Maillard reaction and the like when combined with amino acid. Specifically, the targeted saccharides include those generally used as stabilizers for dry pharmaceutical compositions, particularly pharmaceutical compositions containing a pharmacologically active proteinaceous substance. More specifically, glucose, fructose, maltose and like reducing sugars; sucrose, trehalose, mannitol, dextran 40 and the like are included. These saccharides can be added to a dry composition singularly or in an arbitrary combination. Among those, preferable are sucrose and maltose.

The saccharide content of the dry composition varies depending on the kind of saccharide contained and cannot be generalized. However, a general amount of the saccharide used is 10 wt.% to 90 wt.%, in some cases 20 wt.% to 80 wt.% and in other cases 40 wt.% to 80 wt.% per 100 wt.% of the dry composition. The above proportion is suitably applied when the saccharide used is sucrose.

Content of the hydrophobic amino acid in the dry composition varies depending on the kind of saccharide contained in the dry composition and cannot be generalized. However, per 100 wt.% of the dry composition, the hydrophobic amino acid can be contained generally about 0.1 wt.% to about 70 wt.%, preferably about 0.25 wt.% to about 60 wt.%, more preferably about 0.5 wt.% to about 50 wt.%, and even more preferably about 1 wt.% to about 50 wt.%.

The amount of hydrophobic amino acid used is 0.1 part by weight to 200 parts by weight, preferably about 0.2 part by weight to about 100 parts by weight and more preferably about 1 part by weight to about 100 parts by weight per 100 parts by weight of the saccharide contained in the dry composition.

Dry compositions for which the method of the invention is usable include compositions containing a pharmacologically active proteinaceous substance as an active ingredient.

There is no limitation on the pharmacologically active proteinaceous substances to be used as long as they are pharmacologically effective proteins or peptides (including polypeptides). Examples thereof are enzymes, hemoglobin, immunoglobulins, hormones, blood coagulation factors and like proteins, antiviral polypeptides (ex. interferons-α, -β, -γ, etc.), immunomodulator polypeptides (ex. interleukins-1, 2, 3, 4, 5, 6, 7, 8, etc.), hematinic polypeptides (ex. erythropoietin, granulocyte colony stimulating factor, macrophage-colony stimulating factor and granulocyte-macrophage-colony stimulating factor) and like polypeptides, etc. The proteins and peptides may be contained in the dry composition singularly or in an arbitrary combination. Among these, preferable are interferons.

The above proteins and peptides include those existing in nature, produced by recombinant DNA techniques or chemical syntheses.

The amount of the pharmacologically active proteinaceous substance used varies depending on the kind to be used; however, specific examples of the concentrations thereof are generally not more than 20 wt.%, in some cases 0.00001 wt.% to 10 wt.%, in some cases 0.0001 wt.% to 10 wt.%, in some cases 0.001 wt.% to 10 wt.% and in some cases 0.001 wt.% to 5 wt.%, per 100 wt.% of the dry composition.

The dry compositions for which the method of the invention is used may further contain human serum albumin, a polar amino acid(s) or a salt(s) thereof (ex. an amino acid(s) having a hydropathy index of not larger than 0 or a salt thereof), an inorganic salt(s), gelatin, a surfactant(s), a buffer(s), etc.

The usage of the method of the invention is not limited as long as it is used in the above dry compositions. The hydrophobic amino acid is added to a solution containing saccharide(s) and pharmacologically active proteinaceous substance(s) prior to the preparation of (i.e., before drying) the dry composition (dried product) or mixed into a solution together with saccharide(s) and pharmacologically active proteinaceous substance(s).

Per 100 wt.% of the dry composition, hydrophobic amino acid is generally about 0. 1 wt.% to about 70 wt.%, preferably about 0. 25 wt.% to about 60 wt.%, more preferably about 0.5 wt.% to about 50 wt.%, and even more preferably about 1 wt.% to about 50 wt.%.

The content of hydrophobic amino acid per 100 parts by weight of saccharides contained in the dry composition is 0.1 part by weight to 200 parts by weight, preferably about 0.2 part by weight to about 100 parts by weight, and more preferably about 1 part by weight to about 100 parts by weight.

The invention relates to a method for preventing coloration of dry compositions containing a saccharide comprising use of hydrophobic amino acid. Specific examples of hydrophobic amino acids, saccharides, dry compositions and usage of hydrophobic amino acids are as described above.

Furthermore, the invention provides use of hydrophobic amino acid as a stabilizer. The hydrophobic amino acid suitably functions as a stabilizer when combined with a saccharide and is particularly effective as a stabilizer for dry compositions containing a pharmacologically active proteinaceous substance.

The invention also relates to a dry composition containing a pharmacologically active proteinaceous substance in which a stabilizer composed of hydrophobic amino acid and saccharide is used.

The saccharide(s) used in the invention is not limited but may include those generally used or function as stabilizers for dry pharmaceutical compositions, specifically, pharmaceutical compositions containing a pharmacologically active proteinaceous substance. Specific examples are sucrose, maltose, lactose, trehalose and like disaccharides; mannitol, xylitol and like sugar alcohols; dextran 40, dextran 70; chondroitin sulfuric acid and like polysaccharides. These saccharides can be added to the dry compositions singularly or in an arbitrary combination. Among those, preferable are sucrose, maltose, lactose, trehalose and like disaccharides, and more preferable is sucrose.

The saccharide content of the targeted dry composition may be generally about 10 wt.% to about 90 wt.%, in some cases about 20 wt.% to about 80 wt.%, and in some other cases about 40 wt.% to about 80 wt.% per 100 wt.% of dry composition. The above proportion is suitably applied when sucrose or like disaccharides is used.

The hydrophobic amino acid content in the dry composition may be, per 100 wt.% of dry composition, generally about 0.1 wt.% to about 70 wt.%, preferably about 0.25 wt.% to about 60 wt.%, more preferably about 0.5 wt.% to about 50 wt.%, and even more preferably about 1 wt.% to about 50 wt.%. The proportion of hydrophobic amino acid to saccharide contained in the dry composition is, per 100 parts by weight of saccharide, 0.1 part by weight to 200 parts by weight, preferably about 0.2 part by weight to about 100 parts by weight, and more preferably about 1 part by weight to about 100 parts by weight.

### Examples

The present invention will be described below in more detail with reference to Examples.

### Examples 1 to 3

In each Example, a mixture was prepared so as to contain 0.25 ml of an interferon-α bulk solution (hereinafter referred to as "IFN-α bulk solution"; titer: 2 x 10⁷ IU/ml), 1 mg of Tween 80, 5 mg of sucrose, and 5 mg of an amino acid (isoleucine (Example 1), valine (Example 2), or leucine (Example 3)) per vial (1 ml capacity). A suitable amount of distilled water for injection was added to dissolve them. The mixture was freeze-dried using a shelf-type lyophilizer (LYOVAC GT-4: LEYBOLD). The resultant freeze-dried composition was allowed to stand at 70 °C. The extent of coloration and the titer of the freeze-dried composition were observed with the passage of time, and then the remaining IFN-α activity was estimated by the ratio of the measured IFN-α activity to the IFN-α activity (100%) of the composition immediately after freeze-drying. For comparison, as an amino acid, 5 mg of glycine (Comparative Example 1) or arginine hydrochloride (Comparative Example 2) was used for evaluating the coloration. Furthermore, for evaluating the stability of IFN-α, a test was conducted in the same manner as mentioned above except that a system with no amino acid added was used (Comparative Example 3). The results, including the degree of coloration and the stability of the IFN-α of the compositions obtained in Examples and Comparative Examples, are shown in Table 1 and Table 2.

**Table 1**

| | Preparation | HP-INDEX of Amino Acid | Coloration (70 °C) | | |
|---|---|---|---|---|---|
| | | | Week | Week | Week |
| | | | 0 | 1 | 2 |
| Examples | 1. I FN-α + sucrose + isoleucine | 4.5 | - | - | - |
| | 2. IFN-α + sucrose + valine | 4.2 | - | - | - |
| | 3. IFN-α + sucrose + leucine | 3.8 | - | - | - |
| Comp. Examples | 1. IFN-α + sucrose + glycine | -0.4 | - | + | ++ |
| | 2. IFN-α + sucrose + arginine hydrochloride | -4.5 | - | + | ++ |

| | | | | | |
|---|---|---|---|---|---|
| Evaluation of coloration: - No coloration was observed ± Slight coloration was observed + Little coloration was observed ++ Much coloration was observed +++ Extreme coloration was observed | | | | | |

**Table 2**

| | Preparation | Remaining IFN-α Activity (%) | | |
|---|---|---|---|---|
| | | Week | Week | Week |
| | | 0 | 1 | 2 |
| Examples | 1. IFN-α + sucrose + isoleucine | 100.0 | 71.6 | 51.8 |
| | 2. IFN-α + sucrose + valine | 100.0 | 59.2 | 47.1 |
| | 3. IFN-α + sucrose + leucine | 100.0 | 75.6 | 72.3 |
| Comp. Example | 1. IFN-α + sucrose | 100.0 | 48.6 | 38.7 |

As shown in Table 1, the combined use of sucrose and glycine and the combined use of sucrose and arginine hydrochloride caused coloration of the freeze-dried composition with the passage of time. Using sucrose with hydrophobic amino acid having a hydropathy index of 3.8 or larger prevented coloration of the freeze-dried composition. Furthermore, as shown in Table 2, the stability of IFN-α improved when using sucrose with hydrophobic amino acid having a hydropathy index (HP-INDEX) of 3.8 or larger compared to the stability when using sucrose singularly.

### Examples 4 to 6

In each Example, a mixture was prepared so as to contain 0.25 ml of an IFN-α bulk solution (titer: 2 x 10⁷ IU/ml), 1 mg of Tween 80, 5 mg of maltose, and 5 mg of an amino acid (isoleucine (Example 4), valine (Example 5), or leucine (Example 6)) per vial (1 ml capacity). A suitable amount of distilled water for injection was added to dissolve them. The mixture was freeze-dried using a shelf-type lyophilizer (LYOVAC GT-4: LEYBOLD). The resultant freeze-dried composition was allowed to stand at 70 °C. The extent of coloration of the freeze-dried composition was observed with the passage of time. For comparison, as an amino acid, 5 mg of glycine (Comparative Example 4) or arginine hydrochloride (Comparative Example 5) was used for evaluating the coloration, and the test was conducted in the same manner as mentioned above. The results of the Examples and Comparative Examples are shown in Table 3.

**Table 3**

| | Preparation | HP-INDEX of Amino Acid | Coloration (70 °C) | | |
|---|---|---|---|---|---|
| | | | Week | Week | Week |
| | | | 0 | 1 | 2 |
| Examples | 4. IFN-α + maltose + isoleucine | 4.5 | - | - | - |
| | 5. IFN-α + maltose + valine | 4.2 | - | - | - |
| | 6. IFN-α + maltose + leucine | 3.8 | - | - | - |
| Comp. | 3. IFN-α + maltose + glycine | -0.4 | - | ++ | +++ |
| Examples | 4. IFN-α + maltose + arginine hydrochloride | -4.5 | - | ++ | +++ |

| | | | | | |
|---|---|---|---|---|---|
| Evaluation of coloration: - No coloration was observed ± Slight coloration was observed + Little coloration was observed ++ Much coloration was observed +++ Extreme coloration was observed | | | | | |

As shown in Table 3, the combined use of maltose and glycine and the combined use of maltose and arginine hydrochloride caused coloration of the freeze-dried composition with the passage of time. Using maltose with hydrophobic amino acid having a hydropathy index of 3.8 or larger prevented coloration of the freeze-dried composition.

### Examples 7 to 9

In each Example, a mixture was prepared so as to contain 0.25 ml of an IFN-α bulk solution (titer: 2 x 10⁷ IU/ml), 1 mg of Tween 80, 5 mg of trehalose, and 5 mg of an amino acid (isoleucine (Example 7), valine (Example 8), or leucine (Example 9)) per vial (1 ml capacity). A suitable amount of distilled water for injection was added to dissolve them. The mixture was freeze-dried using a shelf-type lyophilizer (LYOVAC GT-4: LEYBOLD). The resultant freeze-dried composition was allowed to stand at 70 °C. The titer of the freeze-dried composition was observed with the passage of time, and then the remaining IFN-α activity was estimated by the ratio of the measured IFN-α activity to the IFN-α activity (100%) of the composition immediately after freeze-drying. For comparison, the test was conducted in the same manner as mentioned above except that a system with no amino acid added was used (Comparative Example 6) for evaluating the stability of IFN-α. The results of the Examples and Comparative Example are shown in Table 4.

**Table 4**

| Preparation | | Remaining IFN-α Activity (%) | | |
|---|---|---|---|---|
| | | Week | Week | Week |
| | | 0 | 1 | 2 |
| Examples | 7. IFN-α + trehalose + isoleucine | 100.0 | 61.5 | 58.9 |
| | 8. IFN-α + trehalose + valine | 100.0 | 61.5 | 56.9 |
| | 9. IFN-α + trehalose + leucine | 100.0 | 72.7 | 63.0 |
| Comp. Example | 6. IFN-α + trehalose | 100.0 | 39.2 | 28.4 |

As shown in Table 4, the stability of IFN-α improved when using trehalose with hydrophobic amino acid having a hydropathy index (HP-INDEX) of 3.8 or larger compared to the stability when using sucrose singularly.

### Examples 10 to 12

In each Example, a mixture was prepared so as to contain 0.25 ml of an IFN-α bulk solution (titer: 2 x 10⁷ IU/ml), 1 mg of Tween 80, 40 mg of sucrose, and 5 mg of an amino acid (isoleucine (Example 10), valine (Example 11), or leucine (Example 12)) per vial (1 ml capacity). A suitable amount of distilled water for injection was added to dissolve them. The mixture was freeze-dried using a shelf-type lyophilizer (LYOVAC GT-4: LEYBOLD). The resultant freeze-dried composition was allowed to stand at 70 °C. The extent of coloration of the freeze-dried composition was observed with the passage of time. For comparison, as an amino acid, 5 mg of glycine (Comparative Example 7) or arginine hydrochloride (Comparative Example 8) was used for evaluating the coloration, and the test was conducted in the same manner as mentioned above. The results of the Examples and Comparative Examples are shown in Table 5.

**Table 5**

| | Preparation | HP-INDEX of Amino Acid | Coloration (70 °C) | | |
|---|---|---|---|---|---|
| | | | Week | Week | Week |
| | | | 0 | 1 | 2 |
| Examples | 10. IFN-α + sucrose + isoleucine | 4.5 | - | - | - |
| | 11. IFN-α + sucrose + valine | 4.2 | - | - | - |
| | 12. IFN-α + sucrose + leucine | 3.8 | - | - | - |
| Comp. | 7. IFN-α + sucrose + glycine | -0.4 | - | + | ++ |
| Examples | 8. IFN-α + sucrose + arginine hydrochloride | -4.5 | - | + | ++ |

| | | | | | |
|---|---|---|---|---|---|
| Evaluation of coloration: - No coloration was observed ± Slight coloration was observed + Little coloration was observed ++ Much coloration was observed +++ Extreme coloration was observed | | | | | |

As shown in Table 5, the combined use of sucrose and glycine and the combined use of sucrose and arginine hydrochloride caused coloration of the freeze-dried composition with the passage of time. Using sucrose with hydrophobic amino acid having a hydropathy index of 3.8 or larger significantly prevented coloration of the freeze-dried composition.

### Examples 13 to 15

A freeze-dried composition was obtained in the same manner as in Examples 1 to 3 except that 0.25 ml of IFN-β bulk solution (titer: 2 x 10⁷ IU/ml) was used instead of 0.25 ml of IFN-α bulk solution (titer: 2 x 10⁷ IU/ml). The resultant freeze-dried composition was allowed to stand at 70 °C, and the coloration thereof was observed with the passage of time. The results show that, as Examples 1 to 3, coloration of the freeze-dried composition can be prevented by using sucrose with hydrophobic amino acid having a hydropathy index of 3.8 or larger.

### Examples 16 to 18

A freeze-dried composition was obtained in the same manner as in Examples 10 to 12 except that 0.25 ml of IFN-β bulk solution (titer: 2 x 10⁷ IU/ml) was used instead of 0.25 ml of IFN-α bulk solution (titer: 2 x 10⁷ IU/ml). The resultant freeze-dried composition was allowed to stand at 70 °C, and the coloration thereof was observed with the passage of time. The results show that, as Examples 10 to 12, coloration of the freeze-dried composition can be prevented by using sucrose with hydrophobic amino acid having a hydropathy index of 3.8 or larger.

### Examples 19 to 21

A freeze-dried composition was obtained in the same manner as in Examples 1 to 3 except that 0.25 ml of IFN-γ bulk solution (titer: 2 x 10⁷ IU/ml) was used instead of 0.25 ml of IFN-α bulk solution (titer: 2 x 10⁷ IU/ml). The resultant freeze-dried composition was allowed to stand at 70 °C, and the coloration thereof was observed with the passage of time. The results show that, as Examples 1 to 3, coloration of the freeze-dried composition can be prevented by using sucrose with hydrophobic amino acid having a hydropathy index of 3.8 or larger.

### Examples 22 to 24

A freeze-dried composition was obtained in the same manner as in Examples 10 to 12 except that 0.25 ml of IFN-β bulk solution (titer: 2 x 10⁷ IU/ml) was used instead of 0.25 ml of IFN-α bulk solution (titer: 2 x 10⁷ IU/ml). The resultant freeze-dried composition was allowed to stand at 70 °C, and the coloration thereof was observed with the passage of time. The results show that, as Examples 10 to 12, coloration of the freeze-dried composition can be prevented by using sucrose with hydrophobic amino acid having a hydropathy index of 3.8 or larger.

### Examples 25 to 27

A freeze-dried composition was obtained in the same manner as in Examples 1 to 3 except that 0.25 ml of erithropoietin bulk solution (titer: 2 x 10⁷ IU/ml) was used instead of 0.25 ml of IFN-α bulk solution (titer: 2 x 10⁷ IU/ml). The resultant freeze-dried composition was allowed to stand at 70 °C, and the coloration thereof was observed with the passage of time. The results show that, as Examples 1 to 3, coloration of the freeze-dried composition can be prevented by using sucrose with hydrophobic amino acid having a hydropathy index of 3.8 or larger.

### Examples 28 to 30

A freeze-dried composition was obtained in the same manner as in Examples 10 to 12 except that 0.25 ml of erithropoietin bulk solution (titer: 2 x 10⁷ IU/ml) was used instead of 0.25 ml of IFN-α bulk solution (titer: 2 x 10⁷ IU/ml). The resultant freeze-dried composition was allowed to stand at 70 °C, and the coloration thereof was observed with the passage of time. The results show that, as Examples 10 to 12, coloration of the freeze-dried composition can be prevented by using sucrose with hydrophobic amino acid having a hydropathy index of 3.8 or larger.

### Examples 31 to 33

One vial (1 ml capacity) of mixture solution was prepared in such a manner that it comprised 0.25 ml of IFN-α bulk solution (titer: 2 x 10⁷ IU/ml), 40 mg of sucrose, and 5 mg each of two kinds of amino acid (glycine + isoleucine (Example 31), glycine + valine (Example 32), or glycine + leucine (Example 33)). A suitable amount of distilled water for injection was added to dissolve them. Thereafter, freeze-drying was conducted in the same manner as in Examples 10 to 12 obtaining a freeze-dried composition. The resultant freeze-dried composition was allowed to stand at 70 °C. The extent of coloration of the freeze-dried composition was observed with the passage of time. For comparison, the test was conducted in the same manner as mentioned above except that 5 mg of glycine alone (Comparative Example 7) was used as the amino acid. The results of the Examples and Comparative Example are shown in Table 6.

**Table 6**

| | Preparation | HP-INDEX of Amino Acid | Coloration (70 °C) | | |
|---|---|---|---|---|---|
| | | | Week | Week | Week |
| | | | 0 | 1 | 2 |
| Examples | 3.1. IFN-α + sucrose + glycine + isoleucine | 4.5 | - | - | - |
| | 32. IFN-α + sucrose + glycine + valine | 4.2 | - | - | - |
| | 33. IFN-α + sucrose + glycine + leucine | 3.8 | - | - | - |
| Comp. Example | 7. IFN-α + sucrose + glycine | -0.4 | - | + | ++ |

| | | | | | |
|---|---|---|---|---|---|
| Evaluation of coloration: - No coloration was observed ± Slight coloration was observed + Little coloration was observed ++ Much coloration was observed +++ Extreme coloration was observed | | | | | |

As shown in Table 6, the freeze-dried composition containing sucrose and glycine caused coloration with the passage of time. However, coloration of the freeze-dried composition can significantly prevented by using hydrophobic amino acid having a hydropathy index of 3.8 or larger in addition to sucrose and glycin.

### INDUSTRIAL APPLICABILITY

The anti-coloring agent of the invention can significantly reduce and/or prevent the coloration of a dry composition with the passage of time caused by the combined use of saccharide and amino acid. Such an anti-coloring agent is specifically useful for preventing the coloration of a dry composition comprising a saccharide and a pharmacologically active proteinaceous substance. From another point of view, the anti-coloring agent is also useful as a stabilizer for a dry composition containing a saccharide and a pharmacologically active proteinaceous substance. Therefore, the invention can provide dry compositions containing a pharmacologically active proteinaceous substance and an anti-coloring agent or a stabilizer, i.e., dry compositions containing a pharmacologically active proteinaceous substance to which a saccharide and an amino acid are added, which exhibits excellent stability by significantly preventing the coloration and lowered activity observed with the passage of time.

## Claims

1. A method for preventing coloration of a dry pharmaceutical composition containing a saccharide and a pharmacologically active proteinaceous substance, said method comprising
adding hydrophobic amino acid having a hydropathy index of not less than 2 to a solution containing a saccharide and a pharmacologically active proteinaceous substance, wherein said hydrophobic amino acid is added in a total amount of from 0.1 parts by weight to 200 parts by weight per 100 parts by weight of said saccharide,
and drying the solution to obtain said dry composition, thereby preventing coloration of said dry composition.

2. The method for preventing coloration according to claim 1, wherein the hydrophobic amino acid has a hydropathy index of not less than 3.8.

3. The method for preventing coloration according to claim 1, wherein the hydrophobic amino acid has a hydropathy index in the range of from 3.8 to 4.5.

4. The method for preventing coloration according to claim 1, wherein the hydrophobic amino acid is at least one member selected from the group consisting of valine, leucine, isoleucine and phenylalanine.

5. The method for preventing coloration according to claim 1, wherein the saccharide is at least one member selected from the group consisting of reducing sugars, sucrose, trehalose, mannitol and dextran 40.

6. The method for preventing coloration according to claim 1, wherein the hydrophobic amino acid is used in such a manner that its concentration becomes in the range of from 0.1 wt.% to 70 wt.% per 100 wt.% of the total weight of the dry composition.

7. The method for preventing coloration according to claim 1, wherein the dry composition is a freeze-dried composition.

8. The method for preventing coloration according to claim 1, wherein the pharmacologically active proteinaceous substance is at least one member selected from the group consisting of antiviral polypeptides, immunomodulator polypeptides and hematinic polypeptides.

9. The method for preventing coloration according to claim 1, wherein the pharmacologically active proteinaceous substance is interferon.

10. A dry pharmaceutical composition containing a pharmacologically active proteinaceous substance, a saccharide, and hydrophobic amino acid having a hydropathy index of not less than 2, wherein said hydrophobic amino acid is present in a total amount of from 0.1 parts by weight to 200 parts by weight per 100 parts by weight of said saccharide.

11. The dry pharmaceutical composition according to claim 10, wherein the hydrophobic amino acid has a hydropathy index of not less than 3.8.

12. The dry pharmaceutical composition according to claim 10, wherein the hydrophobic amino acid has a hydropathy index in the range of from 3.8 to 4.5.

13. The dry pharmaceutical composition according to claim 10, wherein the hydrophobic amino acid is at least one member selected from the group consisting of valine, leucine, isoleucine and phenylalanine.

14. The dry pharmaceutical composition according to claim 10, wherein the saccharide is at least one member selected from the group consisting of reducing sugars, sucrose, trehalose, mannitol and dextran 40.

15. The dry pharmaceutical composition according to claim 10, wherein the concentration of the hydrophobic amino acid is in the range of from 0.1 wt.% to 70 wt.% per 100 wt.% of the total weight of the dry composition.

16. The dry pharmaceutical composition according to claim 10, which is a freeze-dried composition.

17. The dry pharmaceutical composition according to claim 10, wherein the pharmacologically active proteinaceous substance is at least one member selected from the group consisting of antiviral polypeptides, immunomodulator polypeptides and hematinic polypeptides.

18. The dry pharmaceutical composition according to claim 10, wherein the pharmacologically active proteinaceous substance is interferon.

19. Use of a hydrophobic amino acid having a hydropathy index of not less than 2 for preventing coloration of a dry pharmaceutical composition containing a saccharide, wherein said hydrophobic amino acid is used in a total amount of from 0.1 parts by weight to 200 parts by weight per 100 parts by weight of said saccharide.

20. The use according to claim 19, wherein the hydrophobic amino acid has a hydropathy index of not less than 3.8.

21. The use according to claim 19, wherein the hydrophobic amino acid has a hydropathy index in the range of from 3.8 to 4.5.

22. The use according to claim 19, wherein the hydrophobic amino acid is at least one member selected from the group consisting of valine, leucine, isoleucine and phenylalanine.

23. The use according to claim 19, wherein the saccharide is at least one member selected from the group consisting of reducing sugars, sucrose, trehalose, mannitol and dextran 40.

24. The use according to claim 19, wherein the hydrophobic amino acid is use at a concentration in the range of from 0.1 wt.% to 70 wt.% per 100 wt.% of the total weight of the dry composition.

25. The use according to claim 19, which is a freeze-dried composition.

26. The use according to claim 19, wherein the pharmacologically active proteinaceous substance is at least one member selected from the group consisting of antiviral polypeptides, immunomodulator polypeptides and hematinic polypeptides.

27. The use according to claim 19, wherein the pharmacologically active proteinaceous substance is interferon.

## Patentansprüche

1. Verfahren zur Verhinderung der Färbung einer trockenen pharmazeutischen Zusammensetzung, die ein Saccharid und eine pharmakologisch aktive proteinartige Substanz enthält, wobei das Verfahren folgendes umfaßt:
Zugeben von hydrophober Aminosäure mit einem Hydropathieindex von nicht weniger als 2 zu einer Lösung, die ein Saccharid und eine pharmakologisch aktive proteinartige Substanz enthält, worin die hydrophobe Aminosäure in einer Gesamtmenge von 0,1 bis 200 Gew.Teilen auf 100 Gew.Teile des Saccharids hinzugegeben wird,
und Trocknen der Lösung zum Erhalt der trockenen Zusammensetzung,
wodurch die Färbung der trockenen Zusammensetzung verhindert wird.

2. Verfahren zur Verhinderung der Färbung gemäß Anspruch 1, worin die hydrophobe Aminosäure einen Hydropathieindex von nicht weniger als 3,8 hat.

3. Verfahren zur Verhinderung der Färbung gemäß Anspruch 1, worin die hydrophobe Aminosäure einen Hydropathieindex im Bereich von 3,8 bis 4,5 hat.

4. Verfahren zur Verhinderung der Färbung gemäß Anspruch 1, worin die hydrophobe Aminosäure wenigstens ein Mitglied ist, das aus der Gruppe ausgewählt ist, die aus Valin, Leucin, Isoleucin und Phenylalanin besteht.

5. Verfahren zur Verhinderung der Färbung gemäß Anspruch 1, worin das Saccharid wenigstens ein Mitglied ist, das aus der Gruppe ausgewählt ist, die aus reduzierenden Zuckern, Saccharose, Trehalose, Mannit und Dextran 40 besteht.

6. Verfahren zur Verhinderung der Färbung gemäß Anspruch 1, worin die hydrophobe Aminosäure in einer solchen Weise verwendet wird, daß ihre Konzentration im Bereich von 0,1 bis 70 Gew.% auf 100 Gew.% des Gesamtgewichts der trockenen Zusammensetzung liegt.

7. Verfahren zur Verhinderung der Färbung gemäß Anspruch 1, worin die trockene Zusammensetzung eine gefriergetrocknete Zusammensetzung ist.

8. Verfahren zur Verhinderung der Färbung gemäß Anspruch 1, worin die pharmakologisch aktive proteinartige Substanz wenigstens ein Mitglied ist, das aus der Gruppe ausgewählt ist, die aus antiviralen Polypeptiden, Immunmodulator-Polypeptiden und hämatinischen Polypeptiden besteht.

9. Verfahren zur Verhinderung der Färbung gemäß Anspruch 1, worin die pharmakologisch aktive proteinartige Substanz Interferon ist.

10. Trockene pharmazeutische Zusammensetzung, die eine pharmakologisch aktive proteinartige Substanz, ein Saccharid und eine hydrophobe Aminosäure mit einem Hydropathieindex von nicht weniger als 2 enthält, worin die hydrophobe Aminosäure in einer Gesamtmenge von 0,1 bis 200 Gew.Teilen auf 100 Gew.Teile des Saccharids vorhanden ist.

11. Trockene pharmazeutische Zusammensetzung gemäß Anspruch 10, worin die hydrophobe Aminosäure einen Hydropathieindex von nicht weniger als 3,8 hat.

12. Trockene pharmazeutische Zusammensetzung gemäß Anspruch 10, worin die hydrophobe Aminosäure einen Hydropathieindex im Bereich von 3,8 bis 4,5 hat.

13. Trockene pharmazeutische Zusammensetzung gemäß Anspruch 10, worin die hydrophobe Aminosäure wenigstens ein Mitglied ist, das aus der Gruppe ausgewählt ist, die aus Valin, Leucin, Isoleucin und Phenylalanin besteht.

14. Trockene pharmazeutische Zusammensetzung gemäß Anspruch 10, worin das Saccharid wenigstens ein Mitglied ist, das aus der Gruppe ausgewählt ist, die aus reduzierenden Zuckern, Saccharose, Trehalose, Mannit und Dextran 40 besteht.

15. Trockene pharmazeutische Zusammensetzung gemäß Anspruch 10, worin die Konzentration der hydrophoben Aminosäure im Bereich von 0,1 bis 70 Gew.% auf 100 Gew.% des Gesamtgewichts der trockenen Zusammensetzung ist.

16. Trockene pharmazeutische Zusammensetzung gemäß Anspruch 10, die eine gefriergetrocknete Zusammensetzung ist.

17. Trockene pharmazeutische Zusammensetzung gemäß Anspruch 10, worin die pharmakologisch aktive proteinartige Substanz wenigstens ein Mitglied ist, das aus der Gruppe ausgewählt ist, die aus antiviralen Polypeptiden, Immunmodulator-Polypeptiden und hämatinischen Polypeptiden besteht.

18. Trockene pharmazeutische Zusammensetzung gemäß Anspruch 10, worin die pharmakologisch aktive proteinartige Substanz Interferon ist.

19. Verwendung einer hydrophoben Aminosäure mit einem Hydropathieindex von nicht weniger als 2 zur Verhinderung der Färbung einer trockenen pharmazeutischen Zusammensetzung, die ein Saccharid enthält, worin die hydrophobe Aminosäure in einer Gesamtmenge von 0,1 bis 200 Gew.Teilen auf 100 Gew.Teile des Saccharids verwendet wird.

20. Verwendung gemäß Anspruch 19, worin die hydrophobe Aminosäure einen Hydropathieindex von nicht weniger als 3,8 hat.

21. Verwendung gemäß Anspruch 19, worin die hydrophobe Aminosäure einen Hydropathieindex im Bereich von 3,8 bis 4,5 hat.

22. Verwendung gemäß Anspruch 19, worin die hydrophobe Aminosäure wenigstens ein Mitglied ist, das aus der Gruppe ausgewählt ist, die aus Valin, Leucin, Isoleucin und Phenylalanin besteht.

23. Verwendung gemäß Anspruch 19, worin das Saccharid wenigstens ein Mitglied ist, das aus der Gruppe ausgewählt ist, die aus reduzierenden Zuckern, Saccharose, Trehalose, Mannit und Dextran 40 besteht.

24. Verwendung gemäß Anspruch 19, worin die hydrophobe Aminosäure in einer Konzentration im Bereich von 0,1 bis 70 Gew.% auf 100 Gew.% des Gesamtgewichts der trockenen Zusammensetzung verwendet wird.

25. Verwendung gemäß Anspruch 19, die eine gefriergetrocknete Zusammensetzung ist.

26. Verwendung gemäß Anspruch 19, worin die pharmakologisch aktive proteinartige Substanz wenigstens ein Mitglied ist, das aus der Gruppe ausgewählt ist, die aus antiviralen Polypeptiden, Immunmodulator-Polypeptiden und hämatinischen Polypeptiden besteht.

27. Verwendung gemäß Anspruch 19, worin die pharmakologisch aktive proteinartige Substanz Interferon ist.

## Revendications

1. Procédé pour prévenir la coloration d'une composition pharmaceutique sèche contenant un saccharide et une substance protéinacée pharmacologiquement active, ledit procédé comprenant :
- l'addition d'un acide aminé hydrophobe ayant un indice d'hydropathie non inférieur à 2, à une solution contenant un saccharide et une substance protéinacée pharmacologiquement active, dans laquelle ledit acide aminé hydrophobe est ajouté en une quantité totale de 0,1 partie en poids à 200 parties en poids pour 100 parties en poids dudit saccharide, et
- le séchage de la solution pour obtenir ladite composition sèche, en prévenant ainsi la coloration de ladite composition sèche.

2. Procédé de prévention de coloration selon la revendication 1, dans lequel l'acide aminé hydrophobe a un indice d'hydropathie non inférieur à 3,8.

3. Procédé de prévention de coloration selon la revendication 1, dans lequel l'acide aminé hydrophobe a un indice d'hydropathie de l'ordre de 3,8 à 4,5.

4. Procédé de prévention de coloration selon la revendication 1, dans lequel l'acide aminé hydrophobe est au moins un membre choisi au sein du groupe consistant en la valine, la leucine, l'isoleucine et la phénylalanine.

5. Procédé de prévention de coloration selon la revendication 1, dans lequel le saccharide est au moins un membre choisi au sein du groupe consistant en des sucres réducteurs, saccharose, tréhalose, mannitol et dextran 40.

6. Procédé de prévention de coloration selon la revendication 1, dans lequel l'acide aminé hydrophobe est utilisé de manière telle que sa concentration devienne de l'ordre de 0,1 % en poids à 70 % en poids par rapport aux 100 % en poids du poids total de la composition sèche.

7. Procédé de prévention de coloration selon la revendication 1, dans lequel la composition sèche est une composition lyophilisée.

8. Procédé de prévention de coloration selon la revendication 1, dans lequel la substance protéinacée pharmacologiquement active est au moins un membre choisi au sein du groupe consistant en des polypeptides antiviraux, des polypeptides immunomodulateurs et des polypeptides hématiniques.

9. Procédé de prévention de coloration selon la revendication 1, dans lequel la substance protéinacée pharmacologiquement active est l'interféron.

10. Composition pharmaceutique sèche contenant une substance protéinacée pharmacologiquement active, un saccharide et un acide aminé hydrophobe ayant un indice d'hydropathie non inférieur à 2, dans laquelle ledit acide aminé hydrophobe est présent en une quantité totale de 0,1 partie en poids à 200 parties en poids pour 100 parties en poids dudit saccharide.

11. Composition pharmaceutique sèche selon la revendication 10, dans laquelle l'acide aminé hydrophobe a un indice d'hydropathie non inférieur à 3,8.

12. Composition pharmaceutique sèche selon la revendication 10, dans laquelle l'acide aminé hydrophobe a un indice d'hydropathie de l'ordre de 3,8 à 4,5.

13. Composition pharmaceutique sèche selon la revendication 10, dans laquelle l'acide aminé hydrophobe est au moins un membre choisi au sein du groupe consistant en la valine, la leucine, l'isoleucine et la phénylalanine.

14. Composition pharmaceutique sèche selon la revendication 10, dans laquelle le saccharide est au moins un membre choisi au sein du groupe consistant en des sucres réducteurs, saccharose, tréhalose, mannitol et dextran 40.

15. Composition pharmaceutique sèche selon la revendication 10, dans laquelle la concentration de l'acide aminé hydrophobe est de l'ordre de 0,1 % en poids à 70 % en poids par rapport aux 100 % en poids du poids total de la composition sèche.

16. Composition pharmaceutique sèche selon la revendication 10, qui est une composition lyophilisée.

17. Composition pharmaceutique sèche selon la revendication 10, dans laquelle la substance protéinacée pharmacologiquement active est au moins un membre choisi au sein du groupe consistant en des polypeptides antiviraux, des polypeptides immunomodulateurs et des polypeptides hématiniques.

18. Composition pharmaceutique sèche selon la revendication 10, dans laquelle la substance protéinacée pharmacologiquement active est l'interféron.

19. Utilisation d'un acide aminé hydrophobe ayant un indice d'hydropathie non inférieur à 2, pour prévenir la coloration d'une composition pharmaceutique sèche contenant un saccharide, dans laquelle ledit acide aminé hydrophobe est utilisé en une quantité totale de 0,1 partie en poids à 200 parties en poids pour 100 parties en poids, dudit saccharide.

20. Utilisation selon la revendication 19, dans laquelle l'acide aminé a un indice d'hydropathie non inférieur à 3,8.

21. Utilisation selon la revendication 19, dans laquelle l'acide aminé hydrophobe a un indice d'hydropathie de l'ordre de 3,8 à 4,5.

22. Utilisation selon la revendication, 19, dans laquelle l'acide aminé hydrophobe est au moins un membre choisi au sein du groupe consistant en la valine, la leucine, l'isoleucine et la phénylalanine.

23. Utilisation selon la revendication 19, dans laquelle le saccharide est au moins un membre choisi au sein du groupe consistant en des sucres réducteurs, saccharose, tréhalose, mannitol et dextran 40.

24. Utilisation selon la revendication 19, dans laquelle l'acide aminé hydrophobe est utilisé à une concentration de l'ordre de 0,1 % en poids à 70 % en poids par rapport aux 100 % en poids du poids total de la composition sèche.

25. Utilisation selon la revendication 19, dans laquelle la composition est une composition lyophilisée.

26. Utilisation selon la revendication 19, dans laquelle la substance protéinacée pharmacologiquement active est au moins un membre choisi au sein du groupe consistant en des polypeptides antiviraux, des polypeptides immunomodulateurs et des polypeptides hématiniques.

27. Utilisation selon la revendication 19, dans laquelle la substance protéinacée pharmacologiquement active est l'interféron.
